# EUROPÄISCHE PATENTSCHRIFT

(11) **EP 0 916 366 B1**
(45) Veröffentlichungstag und Bekanntmachung des Hinweises auf die Patenterteilung: **06.05.2004**
(21) Anmeldenummer: 98121192.3
(22) Anmeldetag: 13.11.1998
(51) Int. Cl.: A61N 1/39

(54) **Defibrillator mit Monitor mit drehbarem Bildschirminhalt**
Defibrillator with a monitor having a rotary screen content
Défibrillateur avec un moniteur à contenu rotatif

(30) Priorität: 14.11.1997 DE 19750632
(43) Veröffentlichungstag der Anmeldung: 19.05.1999
(73) Patentinhaber: GE Medical Systems Information Technologies GmbH, 79111 Freiburg i. Br. (DE)
(72) Erfinder: Magin, Thomas, 79279 Vörstetten (DE)
(74) Vertreter: Müller - Hoffmann & Partner

(56) Entgegenhaltungen:
- EP-A- 0 167 122
- EP-A- 0 431 581
- EP-A- 0 609 500
- GB-A- 2 301 201
- US-A- 4 187 858
- US-A- 4 590 943
- US-A- 5 566 098

## Beschreibung

Die vorliegende Erfindung betrifft einen Defibrillator mit integriertem Überwachungsmonitor, auf dessen Bildschirm Vitalparameter eines Patienten anzeigbar sind, mit einer eingebauten Stromversorgungseinrichtung zur netzunabhängigen Stromversorgung und mit einer Anschlußeinrichtung zur externen Stromversorgung des Defibrillators, wie im Oberbegriff der Ansprüche 1 und 2 beschrieben ist.

Ein derartiger Defibrillator ist im Dokument US-A- 4 590 943 offenbart.

Defibrillatoren zur externen oder transthorakalen Defibrillation gehören zur Ausrüstung von Rettungsmitteln, wie insbesondere Rettungsfahrzeugen, und werden so in unterschiedlichen Gebrauchslagen eingesetzt, nämlich einerseits direkt am Einsatzort außerhalb des Rettungsmittels und andererseits während des Transports des Patienten mit dem Rettungsmittel. D. h., der Defibrillator wird beim Eintreffen des Rettungsmittels am Einsatzort aus dem Rettungsmittel entnommen, um für die Behandlung des gewöhnlich am Boden liegenden Patienten herangezogen zu werden. Während des Transports des Patienten mit dem Rettungsmittel zur Klinik wird der Defibrillator ebenfalls benötigt, wozu er aber im Rettungsmittel in eine spezielle Halterung eingesetzt wird.

Bei einem Einsatz außerhalb des Rettungsmittels wird der Defibrillator über seine eigene Stromversorgung, also vorzugsweise einen Akkumulator, versorgt, während der in seine Halterung eingesetzte Defibrillator im Rettungsmittel seine Energie aus dem Bordnetz des Rettungsmittels bezieht, wobei hier der Akkumulator des Defibrillators gleichzeitig aus dem Bordnetz nachgeladen wird.

Ein Defibrillator weist ein im wesentlichen quaderförmiges Gehäuse auf, das bei einer Behandlung eines Patienten außerhalb des Rettungsmittels flach auf dem Boden aufliegt. Im Rettungsmittel wird der Defibrillator aus Platzgründen "hochkantig" in seine Halterung eingesetzt.

Diese unterschiedliche Anordnung des Defibrillators bei einem Einsatz außerhalb des Rettungsmittels und bei einem Einsatz im Rettungsmittel bedingt nun einen Mangel. zu dessen Beseitigung schon die verschiedensten Anstrengungen unternommen wurden.

Ein Defibrillator hat nämlich bekanntlicherweise einen Überwachungsmonitor, auf dem die Vitalparameter eines Patienten angezeigt werden. Bei diesen Vitalparametern handelt es sich zumindest um ein Elektrokardiogramm (EKG) und gegebenenfalls noch weitere Größen. Der Überwachungsmonitor gibt so dem Arzt bzw. Sanitäter eine Rückmeldung über den Erfolg bzw. Nichterfolg des Einsatzes des Defibrillators am Patienten. Eine ständige Beobachtung durch den Arzt bzw. Sanitäter ist also von großer Bedeutung.

Wie aber bereits oben erläutert wurde, befindet sich der Defibrillator beim Einsatz außerhalb des Rettungsmittels und beim Einsatz innerhalb des Rettungsmittels in verschiedenen Lagen, was zwangsläufig auch verschiedene Lagen des Überwachungsmonitors und damit von dessen Bildschirm nach sich zieht.

Dies bedeutet, daß ein Bild, das bei einem Einsatz des Defibrillators außerhalb des Rettungsmittels "normal" auf dem Bildschirm erscheint, bei einem Einsatz innerhalb des Rettungsmittels, wenn sich der Defibrillator in einer Halterung befindet, "auf dem Kopf stehend" erscheint, da der Bildschirm infolge der anderen Lagerung des Defibrillators um 180° gedreht ist. Es braucht nicht betont zu werden, daß die Betrachtung und Auswertung eines "auf dem Kopf stehenden" Bildes für den Arzt bzw. Sanitäter äußerst mühsam ist.

Zur Überwindung dieses Problems wurde bereits ein Defibrillator entwickelt, dessen Bildschirminhalt durch Betätigen eines Bedienungselementes gedreht werden kann. Solche Defibrillatoren haben also zusätzlich zu den medizinisch bedingten Bedienungselementen noch ein weiteres Bedienungselement, das nach Einsetzen des Defibrillators in die Halterung im Rettungsmittel gesondert betätigt und eingestellt werden muß, um die Signaldarstellung auf dem Bildschirm des Überwachungsmonitors in der gewohnten Weise, also "nicht auf dem Kopf stehend", zu erhalten.

Gerade wenn aber ein Patient in ein Rettungsmittel gebracht wird, sind vom Arzt bzw. Sanitäter die verschiedensten notwendigen Handlungen vorzunehmen, so daß das Einstellen eines gesonderten Bedienungselementes am Defibrillator zur Drehung von dessen Bildschirminhalt als störend empfunden wird.

Es ist daher Aufgabe der vorliegenden Erfindung, einen Defibrillator mit einem Überwachungsmonitor mit drehbarem Bildschirminhalt zu schaffen, bei dem ein Arzt oder Sanitäter ohne zusätzliche Handgriffe oder Einstellungen unabhängig vom Einsatzort des Defibrillators außerhalb oder innerhalb eines Rettungsmittels auf dem Überwachungsmonitor des Defibrillators den Bildschirminhalt in richtiger Anordnung und nicht "auf dem Kopf stehend" dargeboten erhält.

Diese Aufgabe wird bei einem Defibrillator der eingangs genannten Art erfindungsgemäß dadurch gelöst, daß der Bildschirminhalt abhängig vom Anliegen der externen Stromversorgung bzw. vom Einsetzen des Defibrillators in eine Halterung automatisch drehbar ist.

Die Erfindung beschreitet also einen überraschend einfachen Weg: mittels eines Fühlers wird abgegriffen, ob die Stromversorgung des Defibrillators über das Bordnetz des Rettungsmittels erfolgt oder der Defibrillator in eine Halterung eingesetzt ist. Wird festgestellt, daß z. B. die Stromversorgung über das Bordnetz des Rettungsmittels vorgenommen wird, so wird der auf dem Überwachungsmonitor des Defibrillators angezeigte Bildschirminhalt um 180° gedreht bzw. umgekehrt. Damit wird erreicht, daß nach Einsetzen des Defibrillators in seine Halterung im Rettungsmittel und nach Kontaktgabe von Kontaktfedern des Defibrillators mit dem Bordnetz des Rettungsmittels bzw. nach Kontaktgabe eines Fühlers der Bildschirminhalt ohne manuellen Eingriff des Arztes bzw. Sanitäters sofort gedreht wird, so daß er wieder in Normallage und "nicht auf dem Kopf stehend" betrachtet werden kann. Dabei sind keine zusätzlichen Handgriffe oder Einstellungen notwendig, so daß auch hierfür die Behandlung des Patienten nicht unterbrochen zu werden braucht.

Die Erfindung ermöglicht so einen speziellen Vorteil, nämlich ein "automatisches" Drehen des Bildschirminhaltes auf den Überwachungsmonitor abhängig vom Einsatzort des Defibrillators, was bisher beim Stand der Technik nicht möglich war.

Als bordnetzunabhängige Stromversorgungseinrichtung des Defibrillators wird in bevorzugter Weise ein Akkumulator verwendet, der bei Anliegen der externen Stromversorgung automatisch aus dem Bordnetz des Rettungsmittels nachladbar ist.

In einer Weiterbildung der Erfindung ist vorgesehen, daß die Drehbarkeit des Bildschirminhaltes in Abhängigkeit von der Stromversorgung bzw. vom Einsatz in die Halterung vor der ersten Inbetriebnahme des Defibrillators aktivierbar ist. D. h., es besteht die Möglichkeit, daß die Drehbarkeit des Bildschirminhaltes nicht aktiviert wird. Dies kann in Spezialfällen zweckmäßig sein, in denen der Defibrillator ausnahmsweise so im Rettungsmittel untergebracht ist, daß sein Überwachungsmonitor in gleicher Lage wie außerhalb des Rettungsmittels ist. In diesem Fall ist eine Drehbarkeit des Bildschirminhaltes unerwünscht, so daß zweckmäßigerweise die Aktivierung der Drehbarkeit nicht vorgenommen wird.

In diesem Zusammenhang sei erwähnt, daß unter Rettungsmitteln allgemein Kraftfahrzeuge, schienengebundene Fahrzeuge, Flugzeuge, Hubschrauber und Wasserfahrzeuge zu verstehen sind, obwohl selbstverständlich Kraftfahrzeuge ganz bevorzugte Anwendungsgebiete darstellen. Steht ausreichend Platz zur Verfügung, so wird der Defibrillator nicht notwendig "hochkantig" in eine Halterung im Rettungsmittel eingesetzt, was bedeutet, daß der Überwachungsmonitor, wie oben erläutert wurde, in normaler Lage seinen Bildschirminhalt wiedergibt.

Eine andere Weiterbildung der vorliegenden Erfindung besteht darin, daß der Bildschirminhalt zusätzlich mittels eines handbetätigten Schalters drehbar ist. Damit wird erreicht, daß in Ausnahmefällen die Drehung des Bildschirminhaltes manuell, also wie bei den oben erwähnten, bereits bestehenden Geräten, vorgenommen werden kann, was etwa dann sinnvoll ist, wenn der Defibrillator im Rettungsmittel nicht aus dessen Bordnetz versorgt werden kann, weil dieses beispielsweise Unterbrechungen zu den Kontaktfedern aufweist.

Schließlich besteht noch eine Weiterbildung der Erfindung in einem den Anschlußeinrichtungen nachgeschalteten Mikrocontroller zur Überwachung der an den Anschlußeinrichtungen liegenden Spannung. Ein solcher Mikrocontroller ist besonders vorteilhaft zur Erfassung der vom Bordnetz des Rettungsmittels gelieferten Spannung.

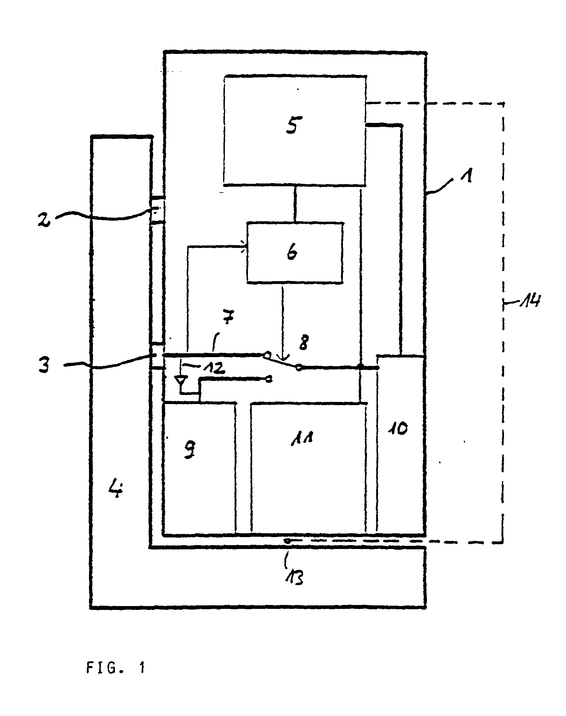

Nachfolgend wird die Erfindung anhand der einzigen Zeichnung näher erläutert, in der ein Blockschaltbild des erfindungsgemäßen Defibrillators schematisch dargestellt ist.

Die Figur zeigt einen Defibrillator 1, der mittels Kontaktfedern 2, 3 an einer Halterung 4 eines nicht dargestellten Rettungsmittels angebracht ist. Ober die Kontaktfeder 2 wird beispielsweise Massepotential zugeführt, während an der Kontaktfeder 3 das Bordnetz mit der Versorgungsspannung von beispielsweise 12 V anliegt.

Im Defibrillator 1 befinden sich ein Hauptmikroprozessor 5 zur Steuerung des Defibrillators insgesamt, ein Mikrocontroller 6, der eine bei Einsatz in die Halterung 4 mit der Kontaktfeder 3 verbundene Eingangsleitung 7 abtastet und abhängig von der erfaßten Spannung einen Schalter 8 steuert. Dieser Schalter 8 ist entweder mit der Eingangsleitung 7 zur versorgung über das Bordnetz oder mit einem Akkumulator 9 verbunden. Anstelle des Akkumulators kann auch beispielsweise eine Batterie verwendet werden.

Es sei angemerkt, daß dieser Akkumulator 9 bei Anschluß des Defibrillators 1 an das Bordnetz nachgeladen wird, was durch eine entsprechende Leitung 12 mit Diode schematisch in der Figur angedeutet ist.

Der Defibrillator 1 enthält weiterhin noch einen Überwachungsmonitor 10 mit einem Bildschirm, beispielsweise eine Flüssigkristallanzeige, sowie einen Hochspannungsteil 11. Der Hochspannungsteil 11 liefert - gesteuert durch den Hauptmikroprozessor 5 - Stromstöße mit einer Zeitdauer von etwa 4 - 8 ms für handtellergroße Elektroden, die an die Brustwand des Patienten anzulegen sind (nicht gezeigt). Auf dem Bildschirm des Überwachungsmonitors 10 werden, wie bereits oben erläutert wurde, die Vitalparameter des Patienten angezeigt.

Wenn der Defibrillator 1 aus der Halterung 4 herausgenommen ist, so stellt der Mikrocontroller 6 fest, daß eine Stromversorgung über die Eingangsleitung 7 nicht mehr erfolgt. Er schaltet dann den Schalter 8 auf den Akkumulator 9 um, so daß dieser den Hauptmikroprozessor 5, den Überwachungsmonitor 10 und den Hochspannungsteil 11 versorgt.

Ist der Defibrillator 1 aus der Halterung 4 herausgenommen, so wird er flach auf den Boden gelegt, um für eine Behandlung eines Patienten in stabiler Lage zu sein. In dieser Lage zeigt der Überwachungsmonitor 10 auf seinem Bildschirm den Bildschirminhalt für einen Arzt bzw. Sanitäter in richtiger Lage an.

Wird nach einer ersten Notbehandlung der Patient sodann zusammen mit dem Defibrillator in das Rettungsmittel gebracht und der Defibrillator in die Halterung 4 eingesetzt, so erscheint der Bildschirminhalt auf dem Überwachungsmonitor 10 an sich "auf dem Kopf stehend". Da jedoch der Mikrocontroller 10 sofort feststellt, daß nach Kontaktgabe über die Kontaktfedern 2, 3 die Stromversorgung aus dem Bordnetz des Rettungsmittels erfolgt, da die Bordspannung von beispielsweise 12 V an der Eingangsleitung 7 anliegt, schaltet er unmittelbar den Schalter 8 auf das Bordnetz um, so daß dieses nunmehr den Defibrillator 1 versorgt. Mit diesem Umschalten wird gleichzeitig über den Mikroprozessor 5 eine Drehung des Bildschirminhaltes auf dem Bildschirm des Überwachungsmonitors 10 um 180° bewirkt, so daß der Bildschirminhalt für den Arzt bzw. Sanitäter nunmehr auch im Rettungsmittel normal abgelesen werden kann.

Der Defibrillator 1 ist so gestaltet, daß die Drehbarkeit des Bildschirminhaltes über den Hauptmikroprozessor 5 in Abhängigkeit von der Stromversorgung über Eingangsleitung 7 bzw. das Bordnetz oder in Abhängigkeit vom Einsatz des Defibrillators in die Halterung vor der ersten Inbetriebnahme des Defibrillators 1 aktivierbar ist. Es sind nämlich Anwendungsfälle denkbar, bei denen eine solche Aktivierung nicht erwünscht ist, wenn beispielsweise der Defibrillator im Rettungsmittel ebenfalls in "flacher Lage" verankert ist. Die Drehbarkeit des Bildschirminhaltes muß also nicht notwendig aktiviert werden.

Außerdem kann noch ein (ebenfalls nicht gezeigter) zusätzlicher Handschalter vorgesehen werden, mit dem die Drehung des Bildschirminhaltes unabhängig von der Stromversorgung manuell vorgenommen werden kann.

Bei dem obigen Ausführungsbeispiel wird der Bildschirminhalt abhängig vom Anliegen der externen Stromversorung (Bordnetz) automatisch gedreht. Diese externe Stromversorgung liegt an, wenn der Defibrillator 1 in die Halterung 4 eingesetzt wird. Daher kann allgemein anstelle des Anliegens der externen Stromversorgung auch das Einsetzen des Defibrillators 1 in die Halterung 4 erfaßt werden, um abhängig davon automatisch die Drehung des Bildschirminhalts vorzunehmen.

Das Einsetzen des Defibrillators 1 in die Halterung 4 kann mittels eines magnetisch oder optisch oder mechanisch oder elektrisch gesteuerten Elementes 13 erfaßt werden, das aktiviert wird, wenn der Defibrillator 1 in die Halterung 4 gelangt. Dieses Einsetzen des Defibrillators 1 in die Halterung 4 wird dem Mikroprozessor 5 gemeldet (vgl. Strichlinie 14), der dann die Drehung des Bildschirminhalts auf dem Überwachungsmonitor 10 vornimmt. Beispiele für das Element 13 sind eine auf Induktion oder einem Magnetfeld beruhende Schalteinrichtung, wie z. B. ein induktiver Näherungsschalter oder ein Reed-Relais, ein einfacher mechanischer Schalter, ein optisches Schaltelement, wie z. B. eine Gabellichtschranke oder ein pyroelektrischer Sensor, ein kapazitiver Schalter oder Schallsensoren jeglicher Art.

## Patentansprüche

1. Defibrillator mit integriertem Überwachungsmonitor (10), auf dessen Bildschirm Vitalparameter eines Patienten anzeigbar sind, mit einer eingebauten Stromversorgungseinrichtung (9) zur netzunabhängigen Stromversorgung und mit einer Anschlußeinrichtung (2, 3, 7) zur externen Stromversorgung des Defibrillators, **dadurch gekennzeichnet, daß** der Bildschirminhalt abhängig vom Anliegen der externen Stromversorgung automatisch drehbar ist.

2. Defibrillator mit integriertem Überwachungsmonitor (10), auf dessen Bildschirm Vitalparameter eines Patienten anzeigbar sind, mit einer eingebauten Stromversorgungseinrichtung (9) zur netzunabhängigen Stromversorgung und mit einer Anschlußeinrichtung (2, 3, 7) zur externen Stromversorgung des Defibrillators, **dadurch gekennzeichnet, daß** der Bildschirminhalt abhängig vom Einsetzen des Defibrillators in eine Halterung automatisch drehbar ist.

3. Defibrillator nach Anspruch 1 oder 2, **dadurch gekennzeichnet, daß** die eingebaute Stromversorgungseinrichtung (9) ein Akkumulator ist, der bei Anliegen der externen Stromversorgung nachladbar ist.

4. Defibrillator nach Anspruch 1, **dadurch gekennzeichnet, daß** die automatische Drehbarkeit des Bildschirminhalts in Abhängigkeit vom Anliegen der externen Stromversorgung vor der ersten Inbetriebnahme des Defibrillators (1) aktivierbar ist.

5. Defibrillator nach einem der Ansprüche 1 bis 4, **dadurch gekennzeichnet, daß** der Bildschirminhalt mittels einer handbetätigten Einrichtung drehbar ist.

6. Defibrillator nach einem der Ansprüche 1 bis 4, **gekennzeichnet durch** einen der Anschlußeinrichtung (2, 3, 7) nachgeschalteten Mikrocontroller (6) zur Überwachung der an der Anschlußeinrichtung (2, 3, 7) liegenden Spannung.

7. Defibrillator nach Anspruch 2, **dadurch gekennzeichnet, daß** das Einsetzen des Defibrillators (1) in der Halterung mittels eines mechanischen oder magnetischen oder optischen oder elektrischen Schalters erfaßbar ist.

## Claims

1. A defibrillator having an integrated surveillance monitor (10), on the screen of which vital parameters of a patient can be displayed, having a built-in power supply device (9) for power supply independently of the main power supply and having a connection device (2, 3, 7) for the external power supply to the defibrillator, **characterized in that** the screen content is automatically rotatable in dependence on the application of the external power supply.

2. A defibrillator having an integrated surveillance monitor (10), on the screen of which vital parameters of a patient can be displayed, having a built-in power supply device (9) for power supply independently of the main power supply and having a connection device (2, 3, 7) for the external power supply to the defibrillator, **characterized in that** the-screen content is automatically rotatable in dependence on the insertion of the defibrillator into a mounting.

3. A defibrillator according to claim 1 or 2, **characterized in that** the built-in power supply device (9) is an accumulator which is rechargeable upon application of the external power supply.

4. A defibrillator according to claim 1, **characterized in that** the automatic rotatability of the screen content can be activated in dependence on the application of the external power supply prior to the first inception of operation of the defibrillator (1).

5. A defibrillator according to one of claims 1 to 4, **characterized in that** the screen content can be rotated by means of a manually actuated device.

6. A defibrillator according to one of claims 1 to 4, **characterized by** a microcontroller (6), connected downstream of the connection device (2, 3, 7), for the surveillance of the voltage present at the connection device (2, 3, 7).

7. A defibrillator according to claim 2, **characterized in that** the insertion of the defibrillator (1) in the mounting can be detected by means of a mechanical or magnetic or optical or electrical switch.

## Revendications

1. Défibrillateur avec un moniteur intégré (10) sur l'écran duquel peuvent être affichés des paramètres vitaux d'un patient, un dispositif d'alimentation intégré (9) pour une alimentation en courant électrique indépendante du secteur, et un dispositif de raccordement (2, 3, 7) pour l'alimentation en courant électrique externe du défibrillateur, **caractérisé en ce que** le contenu d'écran est apte à tourner automatiquement en fonction de l'application de l'alimentation externe.

2. Défibrillateur avec un moniteur intégré (10) sur l'écran duquel peuvent être affichés des paramètres vitaux d'un patient, un dispositif d'alimentation intégré (9) pour une alimentation en courant électrique indépendante du secteur, et un dispositif de raccordement (2, 3, 7) pour l'alimentation en courant électrique externe du défibrillateur, **caractérisé en ce que** le contenu d'écran est apte à tourner automatiquement en fonction de la mise en place du défibrillateur dans une fixation.

3. Défibrillateur selon la revendication 1 ou 2, **caractérisé en ce que** le dispositif d'alimentation intégré (9) est constitué par un accumulateur qui est rechargeable lors de l'application de l'alimentation en courant électrique externe.

4. Défibrillateur selon la revendication 1, **caractérisé en ce que** la possibilité de rotation automatique du contenu d'écran est apte à être activée en fonction de l'application de l'alimentation en courant électrique externe, avant la première mise en marche du défibrillateur (1).

5. Défibrillateur selon l'une des revendications 1 à 4, **caractérisé en ce que** le contenu d'écran est apte à être tourné à l'aide d'un dispositif à commande manuelle.

6. Défibrillateur selon l'une des revendications 1 à 4, **caractérisé par** un microcontrôleur (6) qui est monté en aval du dispositif de raccordement (2, 3, 7) pour surveiller la tension appliquée au niveau de celui-ci.

7. Défibrillateur selon la revendication 2, **caractérisé en ce que** la mise en place du défibrillateur (1) dans la fixation est apte à être détectée à l'aide d'un commutateur mécanique, magnétique, optique ou électrique.
